# EUROPEAN PATENT SPECIFICATION

(11) **EP 1 003 508 B1**
(45) Date of publication and mention of the grant of the patent: **03.05.2006**
(21) Application number: 98940786.1
(22) Date of filing: 05.08.1998
(51) Int. Cl.: A61K 31/455, A61P 35/00

(54) **ANTIMUTAGENIC AGENTS**
ANTIMUTAGENMITTEL
AGENTS ANTIMUTAGENES

(30) Priority: 05.08.1997 US 906466
(43) Date of publication of application: 31.05.2000
(73) Proprietor: The GRASSETTI Family Trust, Jamestown, CA 95327 (US)
(72) Inventor: Grassetti, Davide, R., deceased (US)
(74) Representative: Wibbelmann, Jobst
(86) International application number: PCT/US1998/016262
(87) International publication number: WO 1999/007368

(56) References cited:
- GB-A- 1 416 018
- US-A- 4 378 364
- GRASSETTI D. R.: "Metastases limited" NATURE, vol. 3085, April 1984 (1984-04), page 500 XP002200080
- D'ANCONA S ET AL: "Effects of 6,6'-dithiodinicotinic acid (CPDS) and its metabolite 6-mercaptonicotinic acid (6-MNA) on murine and hamster fibroblasts (3T3 and BHK) and murine metastatic melanoma cells (F10)." CHEMIOTERAPIA: INTERNATIONAL JOURNAL OF THE MEDITERRANEAN SOCIETY OF CHEMOTHERAPY. ITALY AUG 1986, vol. 5, no. 4, August 1986 (1986-08), pages 219-227, XP001076895 ISSN: 0392-906X
- GRASSETTI D R: "CARBOXYPYRIDINE DISULFIDE" DRUGS OF THE FUTURE, vol. 11, no. 7, 1986, pages 559-561, XP008003821 ISSN: 0377-8282
- SUTO M J ET AL: "INHIBITORS OF POLY-ADP-RIBOSE POLYMERASE ADPRP POTENTIAL CHEMOTHERAPEUTIC AGENTS" DRUGS OF THE FUTURE, vol. 16, no. 8, 1991, pages 723-739, XP008003820 ISSN: 0377-8282
- DATABASE MEDLINE [Online] February 1997 (1997-02) KITAHORI Y ET AL: "Genetic alterations in N-bis(2-hydroxypropyl)nitrosamine-induced rat transplantable thyroid carcinoma lines: analysis of the TSH-R, G(alpha)s, ras and p53 genes." Database accession no. NLM9054617 XP002200071 & CARCINOGENESIS. ENGLAND FEB 1997, vol. 18, no. 2, February 1997 (1997-02), pages 265-269, ISSN: 0143-3334
- DATABASE MEDLINE [Online] February 1982 (1982-02) MENG C L ET AL: "A transplantable anaplastic oral cancer model." Database accession no. NLM6799885 XP002200072 & ORAL SURGERY, ORAL MEDICINE, AND ORAL PATHOLOGY. UNITED STATES FEB 1982, vol. 53, no. 2, February 1982 (1982-02), pages 179-189, ISSN: 0030-4220
- DATABASE MEDLINE [Online] October 1996 (1996-10) URBAN T ET AL: "Codon 12 Ki-ras mutation in non-small-cell lung cancer: comparative evaluation in tumoural and non-tumoural lung." Database accession no. NLM8855973 XP002200073 & BRITISH JOURNAL OF CANCER. SCOTLAND OCT 1996, vol. 74, no. 7, October 1996 (1996-10), pages 1051-1055, ISSN: 0007-0920
- DATABASE MEDLINE [Online] March 1990 (1990-03) WANG X ET AL: "[Observation of morphological genesis and histochemistry of pulmonary adenocarcinoma in mice]" Database accession no. NLM2365343 XP002200074 & HUA XI YI KE DA XUE XUE BAO = JOURNAL OF WEST CHINA UNIVERSITY OF MEDICAL SCIENCES = HUAXI YIKE DAXUE XUEBAO / [BIAN JI ZHE, HUA XI YI KE DA XUE XUE BAO BIAN WEI HUI]. CHINA MAR 1990, vol. 21, no. 1, March 1990 (1990-03), pages 54-58, ISSN: 0257-7712
- GRASSETTI, D. R.: "Antimetastatic and antitumor effect of certain heterocyclic disulfides" CURR. CHEMOTHER. IMMUNOTHER., PROC. INT. CONGR. CHEMOTHER., 12TH (1982), MEETING DATE 1981, VOLUME 2, 1365-6. EDITOR(S): PERITI, PIERO;GIALDRONI GRASSI, GIULIANA. PUBLISHER: AM. SOC. MICROBIOL., WASHINGTON, D. C. , XP001077835
- GRASSETTI, D. R.: "The antimetastatic and tumor growth retarding effects of sulfur containing analogs of nicotinamide" CANCER LETT. (SHANNON, IREL.) (1986), 31(2), 187-95 , XP001076897
- GRASSETTI, DAVIDE R.: "Preventing the spread of cancer" CHEM. TECHNOL. (1973), (NOV.), 666-71 , XP001059138
- GRASSETTI D R: "Effect of 6,6'-dithiodinicotinic acid on the dissemination of Ehrlich ascites tumour." NATURE. ENGLAND 17 OCT 1970, vol. 228, no. 268, 17 October 1970 (1970-10-17), pages 282-283, XP001077831 ISSN: 0028-0836
- DATABASE HCAPLUS ON STN, AMERICAN CHEMICAL SOCIETY, AN 1972:94870, GRASSETTI DAVIDE R., "Reversible Blocking of Peripheric Cellular Mercapto Groups by 6,6'-Dithiodinicotinic Acid", XP002915587; & DE,A,2 120 995.

## Description

### BACKGROUND OF THE INVENTION

### 1. Field of the Invention

This invention relates to the inhibition of the mutagenic effects of carcinogens, and in particular, to the use of the compound 6,6' dithiodinicotinic acid in the manufacture of a medicament to inhibit the activity of the enzyme poly(ADP-ribose) polymerase to inhibit the occurrence of mutation-caused cancer, in a human.

### 2. Background of the Invention

Mutations occur in cellular DNA under the effect of ultraviolet light, infrared light, x-rays, ionizing radiation, and chemicals. It is generally accepted that most mutations lead to cancer.

The enzyme poly(ADP-ribose) polymerase (ADPRP) has been suspected to play a regulatory role in many cellular activities, including DNA repair, differentiation, and malignant transformation. Studies of inhibitors of ADPRP have provided evidence of the importance of ADPRP in those cellular activities (M.J. Suto et al., 1991, Drugs of the Future, 16:723-739). Many analogs of nicotinamide, a product of ADPRP's action on NAD, have been studied in the hope of finding a compound that could modulate the activity of ADPRP. It would be desirable to find a selective inhibitor of ADPRP which could inhibit the malignant transformation of human cells *(Ibid).*

### SUMMARY OF THE INVENTION

It has been discovered that 6,6'-dithiodinicotinic acid has an antimutagenic effect. The use of the invention is based on the finding that 6,6'-dithiodinicotinic acid is a useful agent for the prevention of mutations, most of which lead to cancer.

The use of the invention comprises treating a human subject to inhibit the mutagenic effects of carcinogens. The use involves administering to the subject a composition comprising a sufficient amount of 6,6'-dithiodinicotinic acid to inhibit the activity of poly(ADP-ribose) polymerase to thereby inhibit the mutagenic effects, and in particular, their carcinogenic effects. In another aspect, the invention involves a medicament for inhibiting the occurrence of mutations and of cancers derived from mutations, and involves administering to a subject a composition comprising a sufficient amount of 6,6'-dithiodinicotinic acid.

### DETAILED DESCRIPTION OF THE INVENTION

### General Description and Definitions

The practice of the present invention will employ, unless otherwise indicated, conventional molecular and cell biology, cell culture, biochemistry, and organic and medicinal chemical synthesis within the skill of the art. Such techniques are explained fully in the literature. See Cancer Chemotherapy: Principles and Practice, ed. B.A. Chabner, J.M. Collins, Phil., Lippincott Publ., 1990; Cancer: Principles and Practice of Oncology; ed. de Vita, Jr., V.T., Hellman, S., and Rosenberg, S.A., Lippincott Co., Philadelphia, 1993; The Chemotherapy Source Book, ed. Perry, M.C., Williams and Wilkins Publ., Baltimore, 1991). Silverman, Richard B., The Organic Chemistry of Drug Design and Drug Action, Academic Press, Inc. NY (1992); Smith, Michael B., Organic Synthesis, McGraw Hill, Inc., NY, (1994)).

Methods are well known in the art for determining therapeutically effective amounts of the compounds to be used in the present invention. Such methods involve analysis of the pharmaceutical/pharmacokinetic parameters in anti-cancer or antitumor therapy, i.e for inhibiting the growth of cancerous tumors (Wedge. S.R., Porteus, J.K., Newlands, E.S., Cancer Chemother. Pharmacol. (1997) 40:266-272; Legha, S.S., Seminar in Oncology, (1997) 24:S4-24-31; Motzer, R.J., Vogelzang, N.J., Chemotherapy for Renal Cell Carcinoma. In: Raghaven, D., Scher, H.I., Leibel, S.A., et al: eds. Principles and Practice of Genitourinary Oncology, Lippincott-Raven Publ., Philadelphia, pp. 885-96, 1997; Bloom, H.J., Medroxyprogesterone acetate (Provera) in the treatment of metastatic renal cancer, Br. J. Cancer (1971) 25:250-65)

The present invention shows that in the presence of 6,6'-dithiodinicotinic acid the mutagenic effect of carcinogenic chemicals, exemplified by benzo-[a]-pyrene, 2-aflatoxin, and 2-aminofluorene, is greatly diminished.

6,6'-dithiodinicotinic acid (carboxypyridine disulfide, CPDS) (D.R. Grassetti, 1986, Drugs of the Future, 11(7):559-61) is known to prevent metastases in mice (Grassetti, D.R., 1970, Nature 228:282).

Examples 1, 2, and 3 report experiments in which the antimutagenic effects of 6,6'-dithiodinicotinic acid were observed. These experiments were performed with a mutant strain of Salmonella Typhimurium bacteria T-100. When the cells were exposed to varying amounts of benzo-[a]-pyrene, 2-aflatoxin, or 2-aminofluorene, a number of colonies (mutant) were formed. When 6,6'-dithiodinicotinic acid was present in the culture prior to the addition of carcinogen, the number of mutants decreased by about 70%.

As shown in Example 4 below, the use of the present invention is based on the finding that 6,6'-dithiodinicotinic acid inhibited the activity of poly(ADP-ribose) polymerase (ADPRP). Inhibition of ADPRP is useful for blocking the mutagenic effects of carcinogens (Suto and Suto, (1991), Drugs of the Future, 16(8):723-739). It is understood that 6,6'-dithiodinicotinic acid includes alkaline metal salts and alkyl esters.

The present invention provides a pharmaceutical composition comprising an effective amount of 6,6'-dithiodinicotinic acid in pure form or as a pharmaceutically acceptable crude concentrate in association with a pharmaceutical carrier or diluent. Such compositions conveniently contain less than 1% by weight, and preferably 0.2% by weight, of 6,6'-dithiodinicotinic acid, and involve administration of at least 0.1 mg/kilo of body weight of 6,6'-dithiodinicotinic acid. The compositions may be prepared by conventional techniques to be in conventional forms, for example, capsules, tablets, suppositories, dispersible powders, syrups, elixirs, suspensions or solutions for enteral or parenteral administration. Suitable pharmaceutical diluents or carriers include, for example, water, alcohols, natural or hardened oils and waxes, calcium and sodium carbonates, calcium phosphate, kaolin, talc and lactose as well as suitable preserving agents, such as ethyl-p-hydroxybenzoate, suspending agents such as methyl cellulose, tragacanth and sodium alginate, wetting agents such as lecithin, polyoxyethylene stearate and polyoxyethylene sorbitan monooleate, granulating and disintegrating agents such as starch and alginic acid, binding agents such as starch, gelatin and acacia and lubricating agents such as magnesium stearate, stearic acid and talc, in order to provide an elegant and palatable pharmaceutical preparation. Compositions in tablet form may be coated by conventional techniques to delay disintegration of the tablet and absorption of the active ingredient in the gastrointestinal tract and thereby provide sustained action over a long period. Other compounds and methods known in the art for delaying disintegration or for timed-delayed or time-measured delivery of the active ingredients also find use in formulating the active ingredients for use in the methods of the invention. For example, 6,6'-dithiodinicotinic acid may also be combined with liposomes or other delayed-release carrier means to protect the compounds from degradation until they reach their targets and/or facilitate movement of the compounds across tissue barriers.

The preferred compositions from the standpoint of ease of administration are solid compositions, particularly solid-filled gelatin capsules or tablets.

The carcinogens tested here are representative of the carcinogens which form a major part of air pollutants caused by tobacco smoke, the exhaust fumes of internal combustion engines, among other sources.

It has been observed that 6,6'-dithiodinicotinic acid is not a toxic compound. It can be taken in doses of at least approximately 0.1 gram per day for indefinite periods. This fills the requirements for a non-toxic, low-cost chemopreventive agent, which could be administered orally.

### LEGEND TO TABLES 1,2,3

The effect of 6,6'-dithiodinicotinic acid (0.3 mg/plate, in 0.2 M sodium bicarbonate) added to each plate in the Ames test, which is a reverse mutation assay (Ames, B.N. et al, 1975, Mutation Research 31:347) with Salmonella Typhimurium bacteria T-100 was studied. The 6,6'-dithiodinicotinic acid was added to each plate one hour before the addition of the carcinogen. The results reported in the tables represent the average values (number of colonies per plate) of 3 individual determinations.

### EXAMPLE 1

**EFFECTS OF 6,6'-DITHIODINICOTINIC ACID ON MUTAGENESIS BY BENZO-[a]-PYRENE**

| Number of Colonies Per Plate | | | |
|---|---|---|---|
| Benzo-[a]-pyrene (µg/plate) | No Inhibitor | With Inhibitor | Decrease (%) |
| 0 (control) | 100 | 111 | 0 |
| 2.5 | 1286 | 339 | 74 |
| 5.0 | 1171 | 446 | 62 |
| 10.0 | 1130 | 488 | 58 |

### EXAMPLE 2

**EFFECTS OF 6,6'-DITHIODINICOTINIC ACID ON MUTAGENESIS BY 2-AMINOFLUORENE**

| Number of Colonies Per Plate | | | |
|---|---|---|---|
| 2-Aminofluorene (µg/plate) | No Inhibitor | With Inhibitor | Decrease (%) |
| 0 (control) | 149 | 141 | 0 |
| 5.0 | 773 | 705 | 10 |
| 15.0 | 2295 | 655 | 72 |
| 25.0 | 2134 | 791 | 63 |

### EXAMPLE 3

**EFFECTS OF 6,6'-DITHIODINICOTINIC ACID ON MUTAGENESIS BY 2-AFLATOXIN**

| Number of Colonies Per Plate | | | |
|---|---|---|---|
| 2-Aflatoxin (µg/plate) | No Inhibitor | With Inhibitor | Decrease (%) |
| 0 (control) | 108 | 104 | 0 |
| 20 | 504 | 188 | 63 |
| 50 | 1117 | 358 | 68 |
| 100 | 1662 | 471 | 72 |

### EXAMPLE 4

### INHIBITION OF ADPRP BY 6,6'-DITHIODINICOTINIC ACID

ADPRP was prepared from pig thymus using the method of Khan and Shall (Biochem. Soc. Transactions 4:778 (1976)) with various modifications. Enzyme activity was approximately linear up to 15-20 minutes with NAD substrate concentration of 25 µM. Typical maximum enzyme activity was around 0.003 nmol product formed/minute in the assay (i.e. approximately 0.1% of substrate utilized). K_{M} was approximately 25 µM.

Using a standard assay mix containing 2mM dithiothreitol (DTT) and 100 µM crude enzyme/ml incubation, three experiments were performed with 6,6'-dithiodinicotinic acid added at concentrations varying from 5 µg/ml to 600 µg/ml (i.e. 1/60th molar equivalent of DTT to 2 molar equivalents). Significant and specific inhibition of ADPRP by 6,6'-dithiodinicotinic acid was seen at 50 µg/ml with almost complete inhibition at 150 µg/ml and above (i.e. 0.5 molar equivalents of DTT and above). No inhibition was seen at 5 and 25 µg/ml. The addition of excess DTT to an incubation containing 150 µg/ml 6,6'-dithiodinicotinic acid did not increase the extent of conversion of substrate to bound product.

**INHIBITION % OF CONTROLS**

| Concn of 6,6'-dithiodinicotinic acid (µg/ml) | Percent Inhibition at: | | |
|---|---|---|---|
| | 7.5 min. | 15 min. | 20 min. |
| 5 | 0 | 14 | - |
| 25 | 0 | 0 | - |
| 50 | 37 | 38 | - |
| 100 | 68 | 54 | - |
| 150 | 88 | 70 | 90 |
| 300 | - | - | 93 |
| 600 | - | - | 83 |

These are values for individual incubations which were stopped at either 7.5, 15, or 20 minutes. These results indicate that 6,6'-dithiodinicotinic acid is an effective inhibitor of ADPRP, forming the basis of the composition of the invention for treating a human subject to inhibit mutagenic effects of carcinogens, said composition comprising a sufficient amount of 6,6'-dithiodinicotinic acid (or derivatives thereof) to inhibit activity of ADPRP in the subjects in order to inhibit the mutagenic effects of the carcinogens.

## Claims

1. Use of 6,6'-dithiodinicotinic acid in the manufacture of a medicament for the prevention of mutations leading to cancer.

2. Use of Claim 1, wherein said 6,6'dithiodinicotinic acid is to be administered in an amount of at least 0.1 mg/kg of body weight.

## Patentansprüche

1. Verwendung von 6,6'-Dithiodinikotinsäure in der Herstellung eines Medikamentes zur Prävention von Mutationen, die zu Krebs führen.

2. Verwendung nach Anspruch 1, wobei die 6,6'-Dithiodinikotinsäure in einer Menge von mindestens 0,1 mg/kg Körpergewicht zu verabreichen ist.

## Revendications

1. Utilisation de l'acide 6,6'-dithiodinicotinique dans la fabrication d'un médicament pour la prévention de mutations conduisant au cancer.

2. Utilisation selon la revendication 1, dans laquelle ledit acide 6,6'-dithiodinicotinique est à administrer dans une quantité d'au moins 0,1 mg/kg de poids corporel.
